# EUROPEAN PATENT APPLICATION

(11) **EP 3 095 872 A1**
(43) Date of publication of application: **23.11.2016**
(21) Application number: 15168111.1
(22) Date of filing: 19.05.2015
(51) Int. Cl.: C12P 13/00, C12P 13/06, C12N 1/21

(54) **A METHOD OF PRODUCING ALPHA AMINO ACIDS**

(71) Applicant: Evonik Degussa GmbH, 45128 Essen (DE)
(72) Inventor: Haas, Thomas, Dr., 48161 Münster (DE); Bülter, Thomas, Dr., 47279 Duisburg (DE); Gilch, Stefan, Dr., 48249 Dülmen (DE); Thiessenhusen, Anja, 48147 Münster (DE); Dennig, Alexander, Dr., 8010 Graz (AT); Faber, Kurt, Prof., 8042 Graz (AT); Hall, Mèlanie, Dr., 8020 Graz (AT)

(57) **Abstract**

The present invention provides a microbial cell capable of producing at least one alpha amino acid from at least one short chain fatty acid, wherein the cell is genetically modified to comprise
- at least a first genetic mutation that increases the expression relative to the wild type cell of an enzyme E₁ capable of catalysing the hydroxylation of the fatty acid to an alpha-hydroxy fatty acid,
- at least a second genetic mutation that increases the expression relative to the wild type cell of an enzyme E₂ capable of catalysing the oxidation of the an alpha-hydroxy fatty acid to an alpha ketone, and
- at least a third genetic mutation that increases the expression relative to the wild type cell of enzyme E₃ capable of catalysing the oxidation of the alpha ketone to an alpha amino acid wherein the short chain fatty acid is a C₄- C₁₀ fatty acid.

SEQUENCE LISTING

<110>  Evonik Industries AG

<120>  A METHOD OF PRODUCING ALPHA AMINO ACIDS

<130>  201400327

<160>  9

<170>  PatentIn version 3.5

<210>  1
<211>  418
<212>  PRT
<213>  Clostridium acetobutylicum

<400>  1

Met Leu Leu Lys Glu Asn Thr Ala Lys Asp Lys Gly Ile Asp Ser Thr
1               5                   10                  15

Leu Asp Leu Leu Lys Glu Gly Tyr Leu Phe Ile Lys Asn Arg Ala Asp
            20                  25                  30

His Tyr Gln Ser Asp Leu Phe Glu Thr Arg Leu Met Gly Gln Arg Ile
        35                  40                  45

Ile Cys Met Thr Gly Glu Glu Ala Ala Arg Ile Phe Tyr Asp Ser Asp
    50                  55                  60

Lys Phe Lys Arg Gln Gly Ala Ala Pro Lys Arg Val Gln Glu Thr Leu
65                  70                  75                  80

Leu Gly Glu Asn Ala Ile Gln Thr Leu Asp Gly Glu Ser His Leu His
                85                  90                  95

Arg Lys Lys Leu Phe Met Leu Leu Thr Asn Gln Val Gln Gln Lys Arg
            100                 105                 110

Leu Ala Glu Leu Thr Thr Glu Lys Trp Glu Ala Ser Ala Ser Lys Trp
        115                 120                 125

His Thr Lys Ser Ile Val Leu Phe Asn Glu Ala Asn Glu Ile Leu Cys
    130                 135                 140

Gln Val Ala Cys His Trp Ala Gly Val Pro Leu Met Glu Ser Asp Ile
145                 150                 155                 160

Lys Asn Arg Ala Glu Asp Phe Ser Ser Met Ile Asp Ser Phe Gly Ala
                165                 170                 175

Val Gly Pro Arg His Trp Lys Gly Lys Lys Ala Arg Asn Thr Ile Glu
        180                 185                 190

Ala Trp Ile Lys Glu Ile Ile Glu Asn Val Arg Ser Gly Arg Ile Arg
    195                 200                 205

Ala Glu Glu Gly Ser Pro Leu His Glu Ile Ala Phe Tyr Ile Asp Val
    210                 215                 220

Asn Gly Gln Gln Met Pro Ala Glu Met Ala Ala Ile Glu Leu Ile Asn
225                 230                 235                 240

Ile Leu Arg Pro Ile Val Ala Ile Ser Thr Phe Ile Thr Phe Ser Ala
                245                 250                 255

Leu Ala Leu Tyr Glu His Ser Glu Tyr Arg Glu Lys Leu Gln Ser Lys
            260                 265                 270

Asp Ile Arg Tyr Leu Glu Met Phe Thr Gln Glu Val Arg Arg Tyr Tyr
        275                 280                 285

Pro Phe Ala Pro Phe Val Gly Ala Arg Val Arg Lys Asp Phe Leu Trp
    290                 295                 300

Asn Asn Cys Glu Phe Lys Lys Glu Met Leu Val Leu Leu Asp Ile Tyr
305                 310                 315                 320

Gly Thr Asn His Asp Ser Arg Ile Trp Gln Lys Pro Tyr Glu Phe Ile
                325                 330                 335

Pro Asp Arg Phe Arg Ser Tyr Lys Gly Asn Leu Phe Asp Phe Ile Pro
            340                 345                 350

Gln Gly Gly Gly Asp Pro Ser Ser Thr His Arg Cys Pro Gly Glu Gly
        355                 360                 365

Ile Thr Leu Glu Ile Met Lys Thr Ser Leu Asp Phe Leu Ser Thr Lys
    370                 375                 380

Ile Asp Phe Thr Val Pro Asp Gln Asp Leu Ser Tyr Ser Leu Ser Lys
385                 390                 395                 400

Ile Pro Thr Leu Pro Lys Ser Gly Phe Ile Ile Asp Asn Ile Asn Leu
                405                 410                 415

Lys Leu

<210>  2
<211>  417
<212>  PRT
<213>  Bacillus subtilis

<400>  2

Met Asn Glu Gln Ile Pro His Asp Lys Ser Leu Asp Asn Ser Leu Thr
1               5                   10                  15

Leu Leu Lys Glu Gly Tyr Leu Phe Ile Lys Asn Arg Thr Glu Arg Tyr
            20                  25                  30

Asn Ser Asp Leu Phe Gln Ala Arg Leu Leu Gly Lys Asn Phe Ile Cys
        35                  40                  45

Met Thr Gly Ala Glu Ala Ala Lys Val Phe Tyr Asp Thr Asp Arg Phe
    50                  55                  60

Gln Arg Gln Asn Ala Leu Pro Lys Arg Val Gln Lys Ser Leu Phe Gly
65                  70                  75                  80

Val Asn Ala Ile Gln Gly Met Asp Gly Ser Ala His Ile His Arg Lys
                85                  90                  95

Met Leu Phe Leu Ser Leu Met Thr Pro Pro His Gln Lys Arg Leu Ala
            100                 105                 110

Glu Leu Met Thr Glu Glu Trp Lys Ala Ala Val Thr Arg Trp Glu Lys
        115                 120                 125

Ala Asp Glu Val Val Leu Phe Glu Glu Ala Lys Glu Ile Leu Cys Arg
    130                 135                 140

Val Ala Cys Tyr Trp Ala Gly Val Pro Leu Lys Glu Thr Glu Val Lys
145                 150                 155                 160

Glu Arg Ala Asp Asp Phe Ile Asp Met Val Asp Ala Phe Gly Ala Val
                165                 170                 175

Gly Pro Arg His Trp Lys Gly Arg Arg Ala Arg Pro Arg Ala Glu Glu
            180                 185                 190

Trp Ile Glu Val Met Ile Glu Asp Ala Arg Ala Gly Leu Leu Lys Thr
        195                 200                 205

Thr Ser Gly Thr Ala Leu His Glu Met Ala Phe His Thr Gln Glu Asp
    210                 215                 220

Gly Ser Gln Leu Asp Ser Arg Met Ala Ala Ile Glu Leu Ile Asn Val
225                 230                 235                 240

Leu Arg Pro Ile Val Ala Ile Ser Tyr Phe Leu Val Phe Ser Ala Leu
                245                 250                 255

Ala Leu His Glu His Pro Lys Tyr Lys Glu Trp Leu Arg Ser Gly Asn
            260                 265                 270

Ser Arg Glu Arg Glu Met Phe Val Gln Glu Val Arg Arg Tyr Tyr Pro
        275                 280                 285

Phe Gly Pro Phe Leu Gly Ala Leu Val Lys Lys Asp Phe Val Trp Asn
    290                 295                 300

Asn Cys Glu Phe Lys Lys Gly Thr Ser Val Leu Leu Asp Leu Tyr Gly
305                 310                 315                 320

Thr Asn His Asp Pro Arg Leu Trp Asp His Pro Asp Glu Phe Arg Pro
                325                 330                 335

Glu Arg Phe Ala Glu Arg Glu Glu Asn Leu Phe Asp Met Ile Pro Gln
            340                 345                 350

Gly Gly Gly His Ala Glu Lys Gly His Arg Cys Pro Gly Glu Gly Ile
        355                 360                 365

Thr Ile Glu Val Met Lys Ala Ser Leu Asp Phe Leu Val His Gln Ile
    370                 375                 380

Glu Tyr Asp Val Pro Glu Gln Ser Leu His Tyr Ser Leu Ala Arg Met
385                 390                 395                 400

Pro Ser Leu Pro Glu Ser Gly Phe Val Met Ser Gly Ile Arg Arg Lys
                405                 410                 415

Ser

<210>  3
<211>  310
<212>  PRT
<213>  Artificial Sequence

<220>
<223>  L-HIC-DH

<400>  3

Met Ala Arg Lys Ile Gly Ile Ile Gly Leu Gly Asn Val Gly Ala Ala
1               5                   10                  15

Val Ala His Gly Leu Ile Ala Gln Gly Val Ala Asp Asp Tyr Val Phe
            20                  25                  30

Ile Asp Ala Asn Glu Ala Lys Val Lys Ala Asp Gln Ile Asp Phe Gln
        35                  40                  45

Asp Ala Met Ala Asn Leu Glu Ala His Gly Asn Ile Val Ile Asn Asp
    50                  55                  60

Trp Ala Ala Leu Ala Asp Ala Asp Val Val Ile Ser Thr Leu Gly Asn
65                  70                  75                  80

Ile Lys Leu Gln Gln Asp Asn Pro Thr Gly Asp Arg Phe Ala Glu Leu
                85                  90                  95

Lys Phe Thr Ser Ser Met Val Gln Ser Val Gly Thr Asn Leu Lys Glu
            100                 105                 110

Ser Gly Phe His Gly Val Leu Val Val Ile Ser Asn Pro Val Asp Val
        115                 120                 125

Ile Thr Ala Leu Phe Gln His Val Thr Gly Phe Pro Ala His Lys Val
    130                 135                 140

Ile Gly Thr Gly Thr Leu Leu Asp Thr Ala Arg Met Gln Arg Ala Val
145                 150                 155                 160

Gly Glu Ala Phe Asp Leu Asp Pro Arg Ser Val Ser Gly Tyr Asn Leu
                165                 170                 175

Gly Glu His Gly Asn Ser Gln Phe Val Ala Trp Ser Thr Val Arg Val
            180                 185                 190

Met Gly Gln Pro Ile Val Thr Leu Ala Asp Ala Gly Asp Ile Asp Leu
        195                 200                 205

Ala Ala Ile Glu Glu Glu Ala Arg Lys Gly Gly Phe Thr Val Leu Asn
    210                 215                 220

Gly Lys Gly Tyr Thr Ser Tyr Gly Val Ala Thr Ser Ala Ile Arg Ile
225                 230                 235                 240

Ala Lys Ala Val Met Ala Asp Ala His Ala Glu Leu Val Val Ser Asn
                245                 250                 255

Arg Arg Asp Asp Met Gly Met Tyr Leu Ser Tyr Pro Ala Ile Ile Gly
            260                 265                 270

Arg Asp Gly Val Leu Ala Glu Thr Thr Leu Asp Leu Thr Thr Asp Glu
        275                 280                 285

Gln Glu Lys Leu Leu Gln Ser Arg Asp Tyr Ile Gln Gln Arg Phe Asp
    290                 295                 300

Glu Ile Val Asp Thr Leu
305                 310

<210>  4
<211>  429
<212>  PRT
<213>  Artificial Sequence

<220>
<223>  Leucine-DH

<400>  4

Met Glu Leu Phe Lys Tyr Met Glu Thr Tyr Asp Tyr Glu Gln Val Leu
1               5                   10                  15

Phe Cys Gln Asp Lys Glu Ser Gly Leu Lys Ala Ile Ile Ala Ile His
            20                  25                  30

Asp Thr Thr Leu Gly Pro Ala Leu Gly Gly Thr Arg Met Trp Met Tyr
        35                  40                  45

Asn Ser Glu Glu Glu Ala Leu Glu Asp Ala Leu Arg Leu Ala Arg Gly
    50                  55                  60

Met Thr Tyr Ser Asn Ala Ala Ala Gly Leu Asn Leu Gly Gly Gly Lys
65                  70                  75                  80

Thr Val Ile Ile Gly Asp Pro Arg Lys Asp Lys Asn Glu Ala Met Phe
                85                  90                  95

Arg Ala Phe Gly Arg Phe Ile Gln Gly Leu Asn Gly Arg Tyr Ile Thr
            100                 105                 110

Ala Val Asp Val Gly Thr Thr Val Ala Asp Met Asp Ile Ile Tyr Gln
        115                 120                 125

Glu Thr Asp Tyr Val Thr Gly Ile Ser Pro Glu Phe Gly Ser Ser Gly
    130                 135                 140

Asn Pro Ser Pro Ala Thr Ala Tyr Gly Val Tyr Arg Gly Met Lys Ala
145                 150                 155                 160

Ala Ala Lys Glu Ala Phe Gly Ser Asp Ser Leu Glu Gly Lys Val Val
                165                 170                 175

Ala Val Gln Gly Val Gly Asn Val Ala Tyr His Leu Cys Arg His Leu
            180                 185                 190

His Glu Glu Gly Ala Lys Leu Ile Val Thr Asp Ile Asn Lys Glu Val
        195                 200                 205

Val Ala Arg Ala Val Glu Glu Phe Gly Ala Lys Ala Val Asp Pro Asn
    210                 215                 220

Asp Ile Tyr Gly Val Glu Cys Asp Ile Phe Ala Pro Cys Ala Leu Gly
225                 230                 235                 240

Gly Ile Ile Asn Asp Gln Thr Ile Pro Gln Leu Lys Ala Lys Val Ile
                245                 250                 255

Ala Gly Ser Ala Asp Leu Gln Leu Lys Glu Pro Arg His Gly Asp Ile
            260                 265                 270

Ile His Glu Met Gly Ile Val Tyr Ala Pro Asp Tyr Val Ile Asn Ala
        275                 280                 285

Gly Gly Cys Ile Asn Val Ala Asp Glu Leu Tyr Gly Tyr Asn Arg Glu
    290                 295                 300

Arg Ala Met Lys Lys Ile Glu Gln Ile Tyr Asp Asn Ile Glu Lys Val
305                 310                 315                 320

Phe Ala Ile Ala Lys Arg Asp Asn Ile Pro Thr Tyr Val Ala Ala Asp
                325                 330                 335

Arg Met Ala Glu Glu Arg Ile Glu Thr Met Arg Lys Ala Ala Ser Gln
            340                 345                 350

Phe Leu Gln Asn Gly His His Ile Leu Ser Arg Arg Pro Arg Pro Leu
        355                 360                 365

Thr Ala Ala Arg Ala Gly Leu Arg Arg Ala Asp Asp Gly Gly Thr Thr
    370                 375                 380

Thr Met Gln Glu Gln Lys Phe Arg Ile Leu Thr Ile Asn Pro Gly Ser
385                 390                 395                 400

Thr Ser Thr Lys Ile Gly Val Phe Glu Asn Glu Arg Ala Ile Ala Ser
                405                 410                 415

Lys Lys Arg Ser Ala Thr Arg Ala Gly Ala Ser Ala Ile
            420                 425

<210>  5
<211>  333
<212>  PRT
<213>  Artificial Sequence

<220>
<223>  D-HIC-DH

<400>  5

Met Lys Ile Ile Ala Tyr Gly Ala Arg Val Asp Glu Ile Gln Tyr Phe
1               5                   10                  15

Lys Gln Trp Ala Lys Asp Thr Gly Asn Thr Leu Glu Tyr His Thr Glu
            20                  25                  30

Phe Leu Asp Glu Asn Thr Val Glu Trp Ala Lys Gly Phe Asp Gly Ile
        35                  40                  45

Asn Ser Leu Gln Thr Thr Pro Tyr Ala Ala Gly Val Phe Glu Lys Met
    50                  55                  60

His Ala Tyr Gly Ile Lys Phe Leu Thr Ile Arg Asn Val Gly Thr Asp
65                  70                  75                  80

Asn Ile Asp Met Thr Ala Met Lys Gln Tyr Gly Ile Arg Leu Ser Asn
                85                  90                  95

Val Pro Ala Tyr Ser Pro Ala Ala Ile Ala Glu Phe Ala Leu Thr Asp
            100                 105                 110

Thr Leu Tyr Leu Leu Arg Asn Met Gly Lys Val Gln Ala Gln Leu Gln
        115                 120                 125

Ala Gly Asp Tyr Glu Lys Ala Gly Thr Phe Ile Gly Lys Glu Leu Gly
    130                 135                 140

Gln Gln Thr Val Gly Val Met Gly Thr Gly His Ile Gly Gln Val Ala
145                 150                 155                 160

Ile Lys Leu Phe Lys Gly Phe Gly Ala Lys Val Ile Ala Tyr Asp Pro
                165                 170                 175

Tyr Pro Met Lys Gly Asp His Pro Asp Phe Asp Tyr Val Ser Leu Glu
            180                 185                 190

Asp Leu Phe Lys Gln Ser Asp Val Ile Asp Leu His Val Pro Gly Ile
        195                 200                 205

Glu Gln Asn Thr His Ile Ile Asn Glu Ala Ala Phe Asn Leu Met Lys
    210                 215                 220

Pro Gly Ala Ile Val Ile Asn Thr Ala Arg Pro Asn Leu Ile Asp Thr
225                 230                 235                 240

Gln Ala Met Leu Ser Asn Leu Lys Ser Gly Lys Leu Ala Gly Val Gly
                245                 250                 255

Ile Asp Thr Tyr Glu Tyr Glu Thr Glu Asp Leu Leu Asn Leu Ala Lys
            260                 265                 270

His Gly Ser Phe Lys Asp Pro Leu Trp Asp Glu Leu Leu Gly Met Pro
        275                 280                 285

Asn Val Val Leu Ser Pro His Ile Ala Tyr Tyr Thr Glu Thr Ala Val
    290                 295                 300

His Asn Met Val Tyr Phe Ser Leu Gln His Leu Val Asp Phe Leu Thr
305                 310                 315                 320

Lys Gly Glu Thr Ser Thr Glu Val Thr Gly Pro Ala Lys
                325                 330

<210> 6
<211> 42
<212> DNA
<213> Artificial Sequence

<220>
<223> primer R47/Y51 Fw

<220>
<221> misc_feature
<222> (14)..(15)
<223> n is a, c, g, or t

<220>
<221> misc_feature
<222> (26)..(27)
<223> n is a, c, g, or t

<400> 6
cgaggcgcct ggtnnkgtaa cgcgcnnktt atcaagtcag cg

<210>  7
<211>  42
<212>  DNA
<213>  Artificial Sequence

<220>
<223>  primer R47/Y51 Rv


<220>
<221>  misc_feature
<222>  (16)..(17)
<223>  n is a, c, g, or t

<220>
<221>  misc_feature
<222>  (28)..(29)
<223>  n is a, c, g, or t

<400>  7
cgctgacttg ataamnngcg cgttacmnna ccaggcgcct cg                          42


<210>  8
<211>  27
<212>  DNA
<213>  Artificial Sequence

<220>
<223>  primer I401 Fw


<220>
<221>  misc_feature
<222>  (13)..(14)
<223>  n is a, c, g, or t

<400>  8
cagcgtgcgt gtnnkggtca gcagttc                                           27


<210>  9
<211>  27
<212>  DNA
<213>  Artificial Sequence

<220>
<223>  primer I401 Rw


<220>
<221>  misc_feature
<222>  (14)..(15)
<223>  n is a, c, g, or t

<400>  9
gaactgctga ccmnnacacg cacgctg                                           27

## Description

### FIELD OF THE INVENTION

The present invention relates to a biotechnological method of producing alpha amino acids. In particular, the method relates to a biotechnological production of at least one amino acid from a short chain fatty acid.

### BACKGROUND OF THE INVENTION

Amino acids are especially useful as additives in animal feed and as nutritional supplements for human beings. They can also be used in infusion solutions and may function as synthetic intermediates for the manufacture of pharmaceuticals and agricultural chemicals. Some examples of amino acids in industry include, L-glutamic acid used as a flavour enhancer for food, L-lysine and methionine used as large volume additives for animal feed, and L-tryptophan and L-threonine having similar potential applications. L-phenylalanine and L-aspartic acid are used as key components in the manufacture of the sweetener aspartame. Infusion solutions require a range of amino acids including those essential in human diets. Accordingly, amino acids have multiple purposes in a variety of fields and it is thus essential that a safe and efficient means is available for the production of amino acids.

Conventional methods used for the synthesis of amino acids include fermentation, chemical synthesis, extraction from protein hydrolyzates, enzymatic bioconversions and the like. Chemical synthesis of amino acids generally involves the initial formation of a racemic mixture, followed by a chemical or enzymatic resolution of this mixture to yield the optically active product. The chemical resolution is usually by fractional crystallization of the diasteromeric salts of the amino acid. The enzymatic resolution involves using the enzyme L-aminocylase. However, this method usually involves the production of undesired isomers and is not the preferred method for production of amino acids that are to be consumed.

Fermentation methods have the disadvantages of having slow rates of conversion, costly purifications, and very high capital investments. Also, extraction from protein hydrolysates is used only in a few cases because usually the desired amino acid is a relatively low percentage of the total protein. Consequently, enzymatic bioconversion is the ideal method of amino acid production. Enzymatic conversions offer advantages primarily due to reduced capital investments, lower purification costs, and higher rates of conversion. However, the available enzymatic conversations can only function with certain starting substrates. Accordingly, there is always a need in the art for an alternative biotechnological method of producing amino acids from a different starting substrate that works and is efficient.

### DESCRIPTON OF THE INVENTION

The present invention attempts to solve the problems mentioned above by providing a cell that can be used to produce an alpha amino acid from a short chain fatty acid, wherein the short chain fatty acid is a C₄- C₁₀ fatty acid. In particular, the cell may comprise an increased expression relative to the wild type of at least one of the following enzymes:
- enzyme E₁ capable of catalysing the hydroxylation of the fatty acid to an alpha-hydroxy fatty acid;
- enzyme E₂ capable of catalysing the oxidation of the an alpha-hydroxy fatty acid to an alpha ketone; and
- enzyme E₃ capable of catalysing the oxidation of the an alpha ketone to an alpha amino acid.

According to any aspect of the present invention, there is provided a cell capable of producing at least one alpha amino acid from at least one fatty acid, wherein the cell is genetically modified to comprise
- at least a first genetic mutation that increases the expression relative to the wild type cell of an enzyme E₁ capable of catalysing the hydroxylation of the fatty acid to an alpha-hydroxy fatty acid,
- at least a second genetic mutation that increases the expression relative to the wild type cell of an enzyme E₂ capable of catalysing the oxidation of the an alpha-hydroxy fatty acid to an alpha ketone, and
- at least a third genetic mutation that increases the expression relative to the wild type cell of enzyme E₃ capable of catalysing the oxidation of the an alpha ketone to an alpha amino acidwherein the fatty acid is a C₄- C₁₀ fatty acid.

In particular, the enzymes in the cell according to any aspect of the present invention may comprise at least one of the following enzymes: alpha monooxygenase (E₁), a dehydrogenase (E₂), and a leucine dehydrogenase (E₃). More in particular, the cell according to any aspect of the present invention may be capable of increased expression relative to the wild type cell of a monooxygenase (E₁), a dehydrogenase (E₂), and a leucine dehydrogenase (E₃).

The cell according to any aspect of the present invention, allows for a three step process to be carried out in a single pot with no product purification/separation of the polar substrate intermediates (α-hydroxy acid, α-keto acid) required between the stages. Also, another advantage of the cell according to any aspect of the present invention lies in the activity of enzymes E₂ and E₃ being redox-neutral, which saves significant cost of redox equivalents and the reaction equilibrium may be completely shifted towards amino acid production. In particular, enzymes E₂ and E₃ allow for recycling of NAD(P)H co-factor.

An alpha amino acid refers to an amino acid having both the amine and the carboxylic acid groups attached to the first (alpha-) carbon atom. They are known as 2-, alpha-, or α-amino acids and comprise a generic formula of H₂NCHRCOOH wherein R may be an organic substituent. Alpha amino acids include the 23 proteinogenic amino acids. The term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. In particular, the alpha amino acid may be selected from the group consisting of L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine and L-valine. More in particular, the alpha amino acid may be L-leucine. In one example, the alpha amino acid may also be an O-acetyl-L-homoserine.

The term 'short chain fatty acid' used in the present invention refers to a sub-group of fatty acids with aliphatic tails of less than 10 carbons. The fatty acid may be a saturated, unsaturated, branched or unbranched fatty acid. In particular, the short chain fatty acid used according to any aspect of the present invention may be selected from the group consisting of propionic acid, butyric acid/ butanoic acid, valeric acid/ pentanoic acid, caproic acid/ hexanoic acid, enanthic acid/ heptanoic acid, caprylic acid/ octanoic acid, pelargonic acid/ nonanoic acid, capric acid/ decanoic acid and the like. More in particular, the short chain fatty acid used according to any aspect of the present invention may be a C₄- C₁₀ fatty acid. Even more in particular, the fatty acid may be selected from the group consisting of acetic acid, propionic acid, isobutyric acid (2-methylpropanoic acid), butyric acid, isovaleric acid (3-methylbutanoic acid), valeric acid (pentanoic acid), and the like. In one example, the fatty acid may be 4-acetyloxy-butyric acid.

The cell according to any aspect of the present invention may refer to a wide range of microbial cells. In particular, the cell may be a prokaryotic or a lower eukaryotic cell selected from the group consisting of *Pseudomonas, Corynebacterium, Bacillus* and *Escherichia.* In one example, the cell may be *Escherichia coli.* In another example, the cell may be a lower eukaryote, such as a fungus from the group comprising *Saccharomyces, Candida, Pichia, Schizosaccharomyces* and *Yarrowia,* particularly, *Saccharomyces cerevisiae.* The cell may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989).

The cell may be genetically modified to increase the expression relative to the wild type cell of enzyme E₁ capable of catalysing the hydroxylation of the fatty acid to an alpha-hydroxy fatty acid; enzyme E₂ capable of catalysing the oxidation of the an alpha-hydroxy fatty acid to an alpha ketone; and/or enzyme E₃ capable of catalysing the oxidation of the an alpha ketone to an alpha amino acid. E₁ may be an alpha monooxygenase, E₂ may be a dehydrogenase, and/or E₃ may be an amino acid dehydrogenase.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

Any of the enzymes used according to any aspect of the present invention, may be an isolated enzyme. In particular, the enzymes used according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. The enzyme may be enriched by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

The cell and/or enzyme used according to any aspect of the present invention may be recombinant. The term "recombinant" as used herein, refers to a molecule or is encoded by such a molecule, particularly a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme used according to any aspect of the present invention, is recombinant or not has not necessarily implications for the level of its expression. However, in one example one or more recombinant nucleic acid molecules, polypeptides or enzymes used according to any aspect of the present invention may be overexpressed. The term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

The alpha monooxygenase (E₁) used according to any aspect of the present invention may be an enzyme capable of catalyzing the alpha-hydroxylation of fatty acid substrates. In particular, E₁ may be an oxidase selected from the group consisting of rubredoxin-dependent alkane oxidases (EC 1.14.15.3), cytochrome P450 alkane oxidases (EC 1.14.14.1), xylene monooxygenases (EC 1.14.15.-), methane monoxygenases (EC 1.14.13.25), lipoxygenase (EC 1.13.11.-), hydratase (EC 4.2.1.-), 12-hydroxylase (EC 1.14.13.-), diol synthase (EC 1.13.11.- and EC 1.13.12.-), and variants thereof. In particular, the monooxygenase may be capable of oxidising a fatty acid to the respective hydroxy fatty acid. In particular, the monooxygenase may be a cytochrome P450 enzyme. Even more in particular, the cytochrome P450 enzyme may be P450_{Cla} or P450_{BSB} (EC 1.11.2.4). Even more in particular, the monooxygenase used according to any aspect of the present invention may comprise an amino acid sequence that has 50, 55, 60, 65, 70, 75, 80, 85, 90, 95 or 100% sequence identity to SEQ ID NO:1 or 2.

In one example, E₁ may be a cytochrome P450 enzyme that comes in contact with H₂O₂ according to any aspect of the present invention. The application of H₂O₂ and stability of the P450 enzyme in presence of peroxide may be improved with use of an in-situ H₂O₂ producing system. An example of the in-situ H₂O₂ producing system may involve the controlled formation of peroxide in the reaction vessel. In particular, controlled formation of peroxide in the reaction vessel may be carried out using a sensor-controlled electrochemical generation of H₂O₂ (Getrey et al., 2014 and Kreig et al., 2011).

In another example, E₁ may be capable of catalysing O₂-dependent hydroxylation where O₂ may be the sole oxidant. For example, E₁ may be P450_{Cla} and/or P450 _{BSB} (Malca et al., 2011 and Girhard et al., 2007). In one example, E₁ may be a fatty acid hydroxylating peroxygenases from the CYP152-family (Malca et al., 2011). In another example, E₁ may be P450 BM3 monooxygenase (EC 1.14.14.1).

The teachings of the present invention may not only be carried out using biological macromolecules having the exact amino acid or nucleic acid sequences referred to in this application explicitly, for example by name or accession number, or implicitly, but also using variants of such sequences. The term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Thompson *et al.,* 1994, and Katoh *et al.,* 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In one example, the term "variant", with regard to amino acid sequence, comprises, in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In one example, the term "variant" of an amino acid sequence or nucleic acid sequence comprises, in addition to the above degree of sequence identity, any active portion and/or fragment of the amino acid sequence or nucleic acid sequence, respectively, or any nucleic acid sequence encoding an active portion and/or fragment of an amino acid sequence. The term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease may be capable of hydrolysing peptide bonds in polypeptides. The phrase "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, 2, or 1 order of magnitude of the values displayed by the reference molecule with respect to a specific substrate. Similarly, the term "variant" of a nucleic acid comprises nucleic acids the complementary strand of which hybridises, preferably under stringent conditions, to the reference or wild type nucleic acid. A skilled person would be able to easily determine the monooxygenases that will be capable of making hydroxyl fatty acids.

Stringency of hybridisation reactions is readily determinable by one ordinary skilled in the art, and generally is an empirical calculation dependent on probe length, washing temperature and salt concentration. In general, longer probes require higher temperatures for proper annealing, while shorter probes need lower temperatures. Hybridisation generally depends on the ability of denatured DNA to reanneal to complementary strands when present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995). The person skilled in the art may follow the instructions given in the manual "The DIG System Users Guide for Filter Hybridization", Boehringer Mannheim GmbH, Mannheim, Germany, 1993 and in Liebl *et al.,* 1991 on how to identify DNA sequences by means of hybridisation. In one example, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example by lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. The term "homologue" of a nucleic acid sequence, as used herein, refers to any nucleic acid sequence that encodes the same amino acid sequence as the reference nucleic acid sequence, in line with the degeneracy of the genetic code.

According to any aspect of the present invention, the dehydrogenase (E₂) may be capable of catalysing the conversion/ oxidation of an alpha-hydroxy acid to the respective alpha-keto acid. In particular, E₂ may be an alpha-hydroxyacid dehydrogenase (EC 1.1.3.15). More in particular, E₂ may be 2-hydroxyisocaproate dehydrogenase (EC 1.1.1.-) and/or lactate dehydrogenase (EC 1.1.1.28). Even more in particular, the dehydrogenase used according to any aspect of the present invention may comprise an amino acid sequence that has 50, 55, 60, 65, 70, 75, 80, 85, 90, 95 or 100% sequence identity to SEQ ID NO: 3 and/or SEQ ID NO: 5. In another example, E₂ may be an alpha-hydroxy acid oxidase (EC 1.1.3.15), which can catalyse the reaction using O₂ as oxidant (producing H₂O₂). This would be advantageous for the oxidation of α-hydroxyacids 1a-d (scheme 1), as it is irreversible and produces H₂O₂ (Malca et al., 2011) using P₄₅₀ catalysts (Scheme 1). In particular, E₂ may be an FMN-dependent 2-hydroxyacid oxidase (EC 1.1.3.15). More in particular, E₂ may be selected from the group consisting of lactate oxidase (EC 1.1.3.2), (S)-2-hydroxy-acid oxidase (EC 1.1.3.15) and D- or L-mandelate oxidase [EC 1.1.3.X].

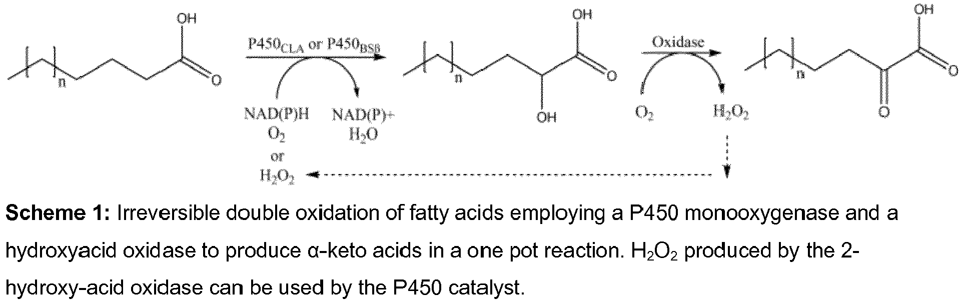

In another example, E₂ may be a monooxygenase where O₂ may be used as an oxidant. In another example, E₂ may be a lactate monooxygenase (EC 1.13.12.4) or a P450 monooxygenase which may be capable of oxidising an alpha-hydroxy acid to the respective alpha-keto acid. In a further example, E₂ may be a dioxygenase (EC 1.13.11) which may be capable of oxidising fatty acids at the alpha-position forming an alpha-hydroperoxy fatty acid, which can then be reduced to yield the alpha-hydroxy acid (Adam, W., 1998 and Adam, W., 1996).

In one example, E₂ may be the same P450 monooxygenase as E₁ leading to double oxidation of the fatty acid to form the respective ketone. In this example, E₁ and E₂ may be the same enzyme and therefore the cell according to any aspect of the present invention may be genetically modified to only increase the expression relative to the wild type cell of two enzymes: E₁ and E₃.

E₃ used according to any aspect of the present invention may be an amino acid dehydrogenase. In particular, the amino acid dehydrogenase may be capable of catalysing the conversion of alpha keto acid, ammonia and NADH to an amino acid, water and NAD+ to. The amino acid dehydrogenase may be an alanine dehydrogenase, i.e. an enzyme capable of catalysing the conversion of pyruvate, ammonia and NADH to L-alanine, water and NAD+. Examples of suitable amino acid dehydrogenases comprise alanine dehydrogenases from *Bacillus subtilis* (accession number: L20916), *Rhizobium leguminosarum* (accession number: CP001622), *Vibrio proteolytikus* (accession number: AF070716), *Mycobacterium tuberculosis* (accession number: X63069) and *Enterobacter aerogenes* (accession number AB013821). In another example, E₃ may be leucine dehydrogenase (EC 1.4.1.9) capable of deamination of L-norleucine and L-leucine. More in particular, the leucine dehydrogenase used according to any aspect of the present invention may comprise an amino acid sequence that has 50, 55, 60, 65, 70, 75, 80, 85, 90, 95 or 100% sequence identity to SEQ ID NO:4. In another example, E₃ may be an ω-transaminase. The ω-transaminase may be selected from any of them as described in Hwang and Kim (2004), Kaulmann et al. (2007), and Yun et al. (2005).

A skilled person would be capable of easily measuring the activity of each of the enzymes E₁, E₂ and E₃. For example, to determine if the expression of E₁ is increased in a cell, a skilled person may use the assay disclosed in Malca S.H, 2011. For example, to determine if the expression of E₂ is increased in a cell, a skilled person may use the assay disclosed in H. Schütte et al, 1984 and Hummel W., et al., 1985. The expression of E₃ in a cell, whether it is increased or decreased, may be measured using the assay disclosed at least in Abrahamson M.J., 2012. A skilled person would easily be able to identify other well-known methods in the art that may be used for measuring the expression of the enzymes used in the cell of the present invention.

In one example, a source of amine may be added to the cell according to any aspect of the present invention to produce at least one alpha amino acid from at least fatty acid. In particular, the source of amines may be any ammonium salt known in the art. More in particular, the amine may be selected from the group consisting of ammonium formate, ammonium chloride, ammonium sulphate and the like. In one example, when E₃ is an alpha transaminase, the source of amine may be alanine. In one example, the following cascade of reactions take place to produce at least one alpha amino acid from at least one short chain fatty acid.

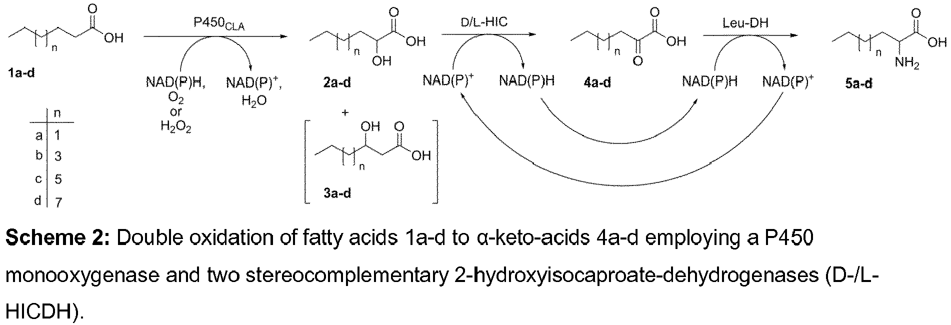

In one specific example, hexanoic acid may be converted to an amino acid, in particular L-Leucine and the three-step process may be carried out in a single pot without isolating the intermediates. In particular, step 1 may comprise the hydroxylation of the fatty acid in α / ß-position using a monooxygenase. In step 2, the α-hydroxy acid may be oxidised by an α-keto acid dehydrogenase (EC 1.2.4.4) (for example, L/D-HIC DHS (EC 1.1.1.337) and an amino acid dehydrogenase (for example leucine-dehdrogenase) to animate the α-amino acid. Stages 2 and 3 are redox-neutral, and the amination necessary cofactor, NAD(P)H is obtained from the oxidation of the α-hydroxy acid.

The cell according to any aspect of the present invention may have reduced capacity of fatty acid degradation by beta-oxidation relative to the wild type cell. In particular, the reduced fatty acid degradation activity compared to the wild type cell may be a result of decreased expression relative to the wild type cell of at least one enzyme selected from the group consisting of acyl-CoA dehydrogenase (FadE) (E₆) (EC:1.3.99.-), enoyl-CoA hydratase (FadB) (E₇) (EC 4.2.1.17), (R)-3-hydroxyacyl-CoA dehydrogenase (FadB) (E₈) (EC 1.1.1.35) and 3-ketoacyl-CoA thiolase (FadA) (E₉) (EC:2.3.1.16).

The term "having a reduced fatty acid degradation capacity", as used herein, means that the respective cell degrades fatty acids, in particular those taken up from the environment, at a lower rate than a comparable cell or wild type cell having normal fatty acid degradation capacity would under identical conditions. In one example, the fatty acid degradation of such a cell is lower on account of deletion, inhibition or inactivation of at least one gene encoding an enzyme involved in the β-oxidation pathway. In one example, at least one enzyme involved in the β-oxidation pathway has lost, in order of increasing preference, 5, 10, 20, 40, 50, 75, 90 or 99 % activity relative to the activity of the same enzyme under comparable conditions in the respective wild type microorganism. The person skilled in the art may be familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., et al, 2007). The phrase "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. It would be within the routine skills of the person skilled in the art to measure the activity of enzymes expressed by living cells using standard essays as described in enzymology text books, for example Cornish-Bowden, 1995.

Degradation of fatty acids is accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalysing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM NAD⁺. The resulting enoyl-CoA is converted to 3-ketoacyl-CoA *via* 3-hydroxylacyl-CoA through hydration and oxidation, catalysed by enoyl-CoA hydratase/(R)-3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and FadI in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., et al, 1997.

The phrase "a cell having a reduced fatty acid degradation capacity", as used herein, refers to a cell having a reduced capability of taking up and/or degrading fatty acids, particularly those having at least eight carbon chains. The fatty acid degradation capacity of a cell may be reduced in various ways. In particular, the cell according to any aspect of the present invention has, compared to its wild type, a reduced activity of an enzyme involved in the β-oxidation pathway. The term "enzyme involved in the β-oxidation pathway", as used herein, refers to an enzyme that interacts directly with a fatty acid or a derivative thereof formed as part of the degradation of the fatty acid via the β-oxidation pathway. The β-oxidation pathway comprises a sequence of reactions effecting the conversion of a fatty acid to acetyl-CoA and the CoA ester of the shortened fatty acid. The enzyme involved in the β-oxidation pathway may by recognizing the fatty acid or derivative thereof as a substrate, converts it to a metabolite formed as a part of the β-oxidation pathway. For example, the acyl-CoA dehydrogenase (EC 1.3.99.-) is an enzyme involved in the β-oxidation pathway as it interacts with fatty acid-CoA and converts fatty acid-CoA ester to enoyl-CoA, which is a metabolite formed as part of the β-oxidation. In another example, the term "enzyme involved in the β-oxidation pathway", as used herein, comprises any polypeptide from the group comprising acyl-CoA dehydrogenase (EC 1.3.99.-), enoyl-CoA hydratase (EC 4.2.1.17), 3-hydroxyacyl-CoA dehydrogenase EC 1.1.1.35) and 3-keto-acyl-CoA thiolase (EC 2.3.1.16). The acyl-CoA synthetase (EC 6.2.1.1) may catalyse the conversion of a fatty acid to the CoA ester of a fatty acid, *i.e.* a molecule, wherein the functional group -OH of the carboxy group is replaced with -S-CoA and introducing the fatty acid into the β-oxidation pathway. For example, the polypeptides FadD and FadK in *E. coli* (accession number: BAA15609.1 and NP_416216.4, respectively) are acyl-CoA dehydrogenases. In one example, the term "acyl-CoA dehydrogenase", as used herein, may be a polypeptide capable of catalysing the conversion of an acyl-CoA to enoyl-CoA, as part of the β-oxidation pathway. For example, the polypeptide FadE in *E. coli* (accession number: BAA77891.2) may be an acyl-CoA dehydrogenase. The term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, as part of the β-oxidation pathway. For example, the polypeptides FadB and FadJ in *E. coli* (accession numbers: BAE77457.1 and P77399.1, respectively) are enoyl-CoA hydratases. The term "ketoacyl-CoA thiolase", as used herein, may refer to a polypeptide capable of catalysing the cleaving of 3-ketoacyl-CoA, resulting in an acyl-CoA shortened by two carbon atoms and acetyl-CoA, as the final step of the β-oxidation pathway. For example, the polypeptides FadA and FadI in *E. coli* (accession number: YP_491599.1and P76503.1, respectively) are ketoacyl-CoA thiolases.

According to another aspect of the present invention, there is provided a method of producing at least one alpha amino acid from at least one short chain fatty acid, the method comprising contacting a recombinant microbial cell with a medium comprising the short chain fatty acid,
wherein the short chain fatty acid is a C₄- C₁₀ fatty acid; and
wherein the microbial cell is genetically modified to comprise
- at least a first genetic mutation that increases the expression relative to the wild type cell of an enzyme E₁ capable of catalysing the hydroxylation of the fatty acid to an alpha-hydroxy fatty acid,
- at least a second genetic mutation that increases the expression relative to the wild type cell of an enzyme E₂ capable of catalysing the oxidation of the an alpha-hydroxy fatty acid to an alpha ketone, and
- at least a third genetic mutation that increases the expression relative to the wild type cell of enzyme E₃ capable of catalysing the oxidation of the an alpha ketone to an alpha amino acid.

The term "contacting", as used herein, means bringing about direct contact between the amino acid, the fatty acid and/or the cell according to any aspect of the present invention in an aqueous solution. For example, the cell, the amino acid and the fatty acid may not be in different compartments separated by a barrier such as an inorganic membrane. If the amino acid or fatty acid is soluble and may be taken up by the cell or can diffuse across biological membranes, it may simply be added to the cell according to any aspect of the present invention in an aqueous solution. In case it is insufficiently soluble, it may be solved in a suitable organic solvent prior to addition to the aqueous solution. The person skilled in the art is able to prepare aqueous solutions of amino acids or fatty acids having insufficient solubility by adding suitable organic and/or polar solvents. Such solvents may be provided in the form of an organic phase comprising liquid organic solvent. In one example, the organic solvent or phase may be considered liquid when liquid at 25 °C and standard atmospheric pressure. In another example, a fatty acid may be provided in the form of a fatty acid ester such as the respective methyl or ethyl ester. In another example, the compounds and catalysts may be contacted *in vitro,* i.e. in a more or less enriched or even purified state, or may be contacted *in situ,* i.e. they are made as part of the metabolism of the cell and subsequently react inside the cell.

The term "an aqueous solution" or "medium" comprises any solution comprising water, mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli.* It is advantageous to use as an aqueous solution a minimal medium, *i.e.* a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

According to any aspect of the present invention, the fatty acid is added to an aqueous solution comprising the cell according to any aspect of the present invention. This step may not only comprise temporarily contacting the fatty acid with the solution, but in fact incubating the fatty acid in the presence of the cell sufficiently long to allow for an oxidation reaction and possible further downstream reactions to occur, for example for at least 1 , 2, 4, 5, 10 or 20 hours. The temperature chosen must be such that the inventive cells remains catalytically competent and/or metabolically active, for example 10 to 42 °C, preferably 30 to 40 °C, in particular, 32 to 38 °C in case the cell is an *E. coli* cell.

In one example, the method according to any aspect of the present invention may comprise a further step of adding a "water-immiscible organic solvent" into the mixture of the cell according to any aspect of the present invention and fatty acids either following step (a) or simultaneously with step (a). The person skilled in the art knows numerous water-immiscible organic solvents that may be used according to any aspect of the present invention. In one example, the term "water-immiscible organic solvent", as used herein, refers to a compound comprising at least two carbon atoms and having the tendency to form, in the presence of an aqueous liquid phase, at 25 °C, another liquid phase, which is clearly separate from the aqueous phase. The separate phase may be a continuous liquid phase or an emulsion. In on example, the term "water-immiscible", as used herein, refers to the tendency of a liquid compound not to be soluble in the water. In another example, the term "water-immiscible", as used herein, means that a compound designated as such has a pH-value (J Sangster, 1997) the decadic logarithm of which exceeds 0, 0.5, 1 or 2. Examples of water-immiscible organic solvents include, but are not limited to water-immiscible solvents from the group comprising substituted and linear alkanes liquid at room temperature, cycloalkanes, cycloalkenes, aryls, fatty acids, fatty acid esters, alcohols, heterocycloalkanes, heterocycloalkenes and heteroaryls. The water-immiscible organic solvents may comprise more than one organic solvent. In a preferred embodiment, the term "extracting" a product using a "water-immiscible organic solvent", as used herein, means that the aqueous solution comprising the cell according to any aspect of the present invention is contacted with the water-immiscible organic solvent sufficiently long as to allow the product to enter the phase comprising the water-immiscible solvent. Subsequently, the phase comprising the water-immiscible organic solvent may be separated from the aqueous solution, for example by distillation or by decantation.

In one example, the water-immiscible organic solvent may be a fatty acid or an ester thereof, in one example, the fatty acid is represented by the formula CH₃ - (CH₂)ₓ - COORs, wherein x is 8, 9, 10, 28 and is more preferably 12 or more than 12, and wherein Rs is H, or alkyl, the latter may be methyl or ethyl. In one example, the water-immiscible organic solvent may be an unsaturated fatty acid, one having a carbon-carbon double bond at position 9 of the carbon chain, or one having 12 carbon atoms or more. In another example, the water-immiscible organic solvent may be oleic acid or hexanoic acid or lauric acid methyl ester. The volume of the water-immiscible organic solvent is such that it is straightforward to separate it from the aqueous solution. In one example, the volume of the water-immiscible organic solvent is 2 to 98, 5 to 95, 10 to 40, or 20 to 30 percent of the total combined volumes of aqueous solution and water-immiscible organic solvent.

In particular, the cofactor of the method according to any aspect of the present invention may be NAD+/NADH. More in particular, the method further comprises a coupled process of cofactor regeneration for regenerating the consumed cofactor NAD(P)+. The coupled cofactor regenerating process also comprises the regeneration of the consumed sacrificial alpha-keto acid. Thus, if cofactor regeneration and regeneration of sacrificial alpha-keto acid are coupled to the amination process merely sub-stoichiometric, or catalytic, amounts of cofactor and fatty acids are required. In one example, wherein regeneration of said sacrificial alpha-keto acid shall not take place, the acid is applied in at least stoichiometric amounts, or even at a molar excess of said acid may be of advantage.

### BRIEF DESCRIPTION OF FIGURES

The inventions are further illustrated by the following figures and non-limiting examples from which further embodiments, aspects and advantages of the present invention may be taken.
Figure 1 is the results of GC-Chromatogram and MS-spectra for 2-hydroxydecanoic acid (rt 8.2 min) after purification and esterification.
Figure 2 is a graph showing the results of oxidation of 2-hydroxyhexanoic (2-OH-C6) and 2-hydroxyotcanoic acid (2-OH-C8) by L-/D-HICDH. Following NADH concentrations were estimated after 1 min reaction time: 2-OH-C6 (1 mM): 45 µM; 2-OH-C6 (10 mM): 142 µM; 2-OH-C8 (1 mM): 41 µM; 2-OH-C8 (10 mM): 126µM.
Figure 3 is a graph showing the oxidation of L-leucine and L-norleucine using Leu-DH.
Figure 4 is the results of a GC-MS chromatogram after one-pot three-step cascade conversion of 10 mM hexanoic acid and derivatization (upper; black line). The lower chromatogram (blue line) shows derivatized sample of 4 mg of L-norleucine (commercial standard; -8.9 min).
Figure 5 is a graph showing: A: Reaction with 2b containing D-/L-HICDH and YcnD; B: Analytical standard of 4b; C: Analytical standard of 2b.
Figure 6 is an LC-MS scan for mass 190.08+1 after conversion of EAB with P450 BM3 variant M2. a = product peak 1 with mass 191. b = product peak 2 with mass 191.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### α-Hydroxylation of selected fatty acids and kinetic characterization

Hydroxylation of selected fatty acids was carried out using a fresh preparation of purified P450_{Cla}. P450_{Cla} was characterised for key catalytic parameters specific activities were determined for three selected substrates as shown in Table 1.

**Table 1: Catalytic and kinetic characterization of P450_{Cla} for conversion of 10 mM of selected fatty acids. All reactions were performed in triplicate.**

| **Substrate** | **Time [min]** | **H₂O₂ [mM]** | **Product [mM]** | **Coupling* [%]** | **Selectivity [% α-OH]** | **TTN [-]** | **TON [min⁻¹]** | **U mg^{-1§}** |
|---|---|---|---|---|---|---|---|---|
| C10 | 5 | 0.8 | 0.67 ± 0.04 | 83 | >95 % | 1330 | 266 | 5.5 |
| C10 | 15 | 0.8 | 0.6 ± 0.02 | 75 | >95 % | 1207 | 80 | 1.7 |
| C10 | 30 | 1.6 | 1.2 ± 0.06 | 75 | >95 % | 2406 | 80 | 1.7 |
| C8 | 5 | 0.8 | 0.49 ± 0.02 | 61 | >95 % | 488 | 98 | 2.0 |
| C8 | 15 | 0.8 | 0.51 ± 0.01 | 63 | >95 % | 506 | 34 | 07 |
| C8 | 30 | 1.6 | 0.94 ± 0.05 | 59 | >95 % | 938 | 31 | 0.7 |
| C6 | 5 | 0.8 | 0.69 = 0.06 | 87 | >95 % | 695 | 139 | 2.9 |
| C6 | 15 | 0.8 | 0.71 = 0.03 | 88 | >95 % | 706 | 47 | 1.0 |
| C6 | 30 | 1.6 | 0.88 = 0.07 | 55 | >95 % | 880 | 29 | 0.6 |

Conditions: 0.5 µM P450_{Cla} used in reactions with C10; 1 µM P450_{Cla} used in reactions with C8 and C6; All reactions were performed in Tris-HCl (pH 7.5, 0.1 M) at RT and 170 rpm shaking in closed glass vials; *mol_{Product}⁻¹ mol_{H2O2}⁻¹; ^{§}molecular weight of P450_{Cla} is 48 kDa, values only relevant at initial rate.

Results showed that P450_{Cla} displayed the desired selectivity (>90 % α-OH) as well as specific activity for all three substrates with a chain-length of C6, C8 and C10 (≥1 U mg⁻¹).

### Preparative scale conversion of decanoic acid, product isolation and purification

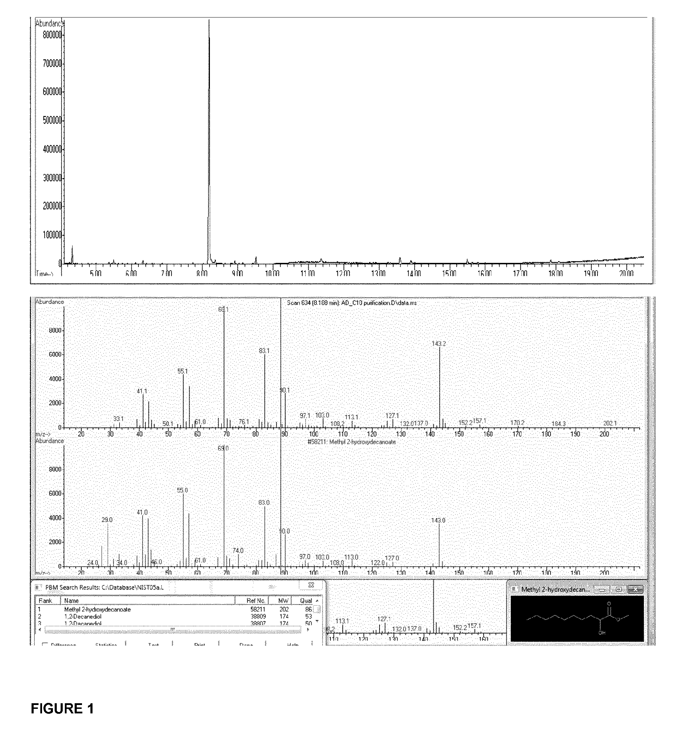

Synthesis of 2-hydroxydecanoic acid was done in 10 ml Tris-HCl (pH 7.5; 0.1 M) employing 4µM P450_{Cla}, 20 mM decanoic acid (38.8 mg of the sodium salt) and 2.5 % EtOH (RT, 170 rpm shaking). Hydrogen peroxide (1.6 mM) was added every 30 min (20 times; in total 32 mM) to the reaction mixture. After 24 h reaction time, 1 ml 5 N HCl was added to stop the reaction. Product extraction was repeated five times using 5 ml EtOAc prior to purification by silica chromatography column using EtOAc/petroleum ether/acetic acid as mobile phase (80:20:1.2). Fractions containing the desired product were combined and concentrated by evaporation under reduced pressure. With the present set up, 16 mg of 2-hydroxydecanoic acid (46 % isolated yield, TTNP450 ∼ 3040) was isolated which was confirmed by GC-MS, 1 H- and 13C-NMR (Figure 1).

The purified compound was used for catalytic characterization of P450_{Cla} in the conversion of decanoic acid (Table 1).

### Dehydrogenation of α-Hydroxy acid with Dehydrogenases- Oxidation of α-hydroxy acids with L-and D-HICDH

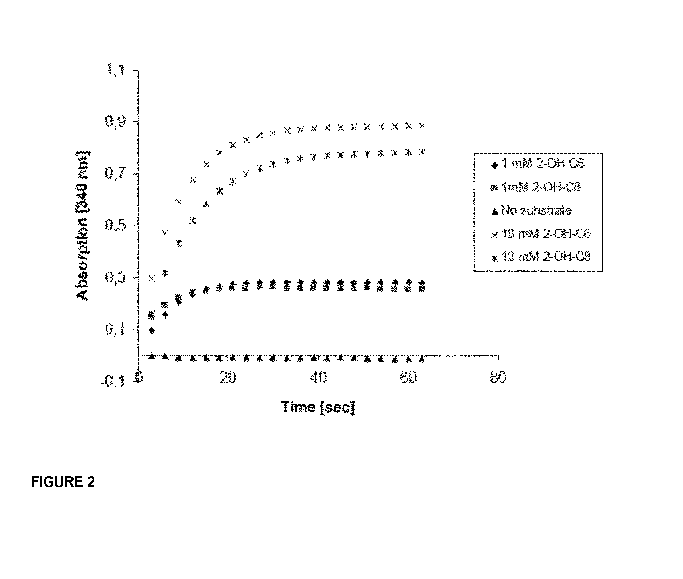

A modified protocol for oxidation of 2-hydroxy acids with L-and D-HICDH was established allowing oxidation of 2-hydroxyhexanoic acid and 2-hydroxyoctanoic acid (both commercial standards) that are produced in step 1 of the cascade (Scheme 1). Reactions contained 1 or 10 mM substrate, 1 % DMSO, 10 mM NAD+ and 0.25 mg of L- and D-HICDH (cell free freeze-dried lysates) in a final volume of 1 ml Tris-HCl (pH 8.5, 0.1 M). Oxidation of the α-hydroxy acid substrates was successful as indicated by increasing UV absorption at 340 nm, corresponding to (concomitant) formation of NADH (Figure 2).

### Dehydrogenation of α-Hydroxy acid with Dehydrogenases- Deamination of L-norleucine and L-leucine using leucine-DH

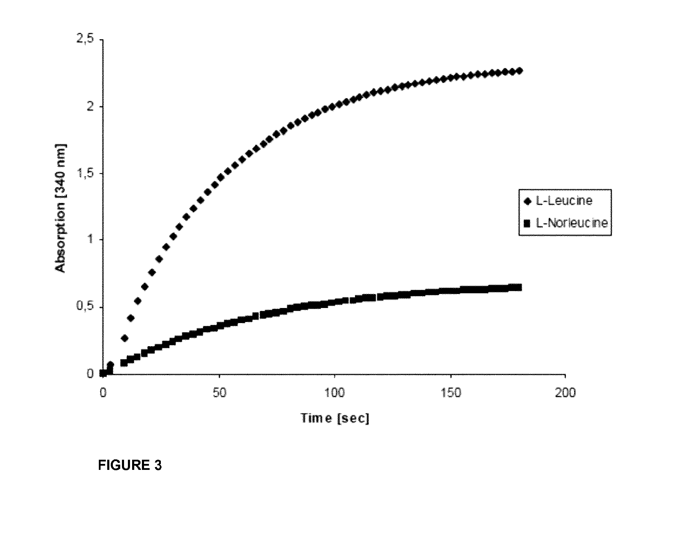

Deamination of L-norleucine and L-leucine (both commercial standards) was performed using a purified leucine-DH. Reactions contained 10 mM substrate, 10 mM NAD+ and 5 µl leucine-DH in a final volume of 1 ml Tris-HCl (pH 8.5, 0.1 M). Oxidation of the amino acids was successful as indicated by increasing UV absorption at 340 nm, corresponding to (concomitant) formation of NADH (Figure 3).

### EXAMPLE 2

P450_{BSß} was expressed and purified as described in Matsunaga et al. 2001. P450_{Cla} was ordered as synthetic gene and cloned into the pET28a expression vector. Efficient expression of P450_{Cla} was obtained by applying the expression protocol (TBmedia, 20 h, 30 °C) as described in Nazor et al. (2008) under heading 'shake flask expression of P450 BM3'. Purification was performed according to the published protocol (Girhard M., et al, 2007) with minor alterations as (unreported) precipitation of P450_{Cla} during dialysis occured. The dialysis buffer was supplemented with 300 mM NaCl to prevent precipitation of P450_{Cla}. Next, hydroxylation of fatty acid substrates 1a-d (scheme 2) was performed employing purified P450_{Cla} and P450_{BSß}. The results are summarized in Table 2. The oxidant source was varied in case of P450_{Cla} while only H₂O₂ was used with P450_{BSß}.

**Table 2: Conversion of fatty acid substrates 1a-d with P450_{Cla} and CYP_{BSß}**

| **Catalyst** | **Oxidant** | **Substrate** | **Concentration [mM]** | **Conversion [%]** | **2a-d [mM]** | **α/β-Regioselectivity (2a-d vs 3a-d)** |
|---|---|---|---|---|---|---|
| P450_{Cla} | H₂O₂ | **1d** | 10 | >99^{a} | n.q.^{d} | |
| P460_{Cla} | H₂O₂ | **1d** | 5 | >99^{a} | n.q.^{d} | |
| P450_{Cla} | H₂O₂ | **1c** | 10 | >99^{a} | n.q.^{d} | |
| P450_{Cla} | H₂O₂ | **1c** | 5 | >99^{a} | n.q.^{d} | |
| P450_{Cla} | H₂O₂ | **1b** | 10 | >99 | 10^{c} | **2a-d** >95%^{e} |
| P450_{Cla} | H₂O₂ | **1b** | 5 | 96 | 4.8 | |
| P450_{Cla} | H₂O₂ | **1a** | 10 | 51 | 5.1^{c} | |
| P450_{Cla} | H₂O₂ | **1a** | 5 | 62 | 3.1 | |
| P450_{Cla}+CamAB | O₂ + NADPH | **1d** | 10 | 5^{b} | n.q.^{d} | |

| **Catalyst** | **Oxidant** | **Substrate** | **Concentration [mM]** | **Conversion [%]** | **2a-d [mM]** | **α/β-Regioselectivity (2a-d vs 3a-d)** |
|---|---|---|---|---|---|---|
| P450_{Cla} | O₂ + NADPH | **1d** | 10 | 0 | n.q.^{d} | ------ |
| CYP_{BSß} | H₂O₂ | **1c** | 10 | 46^{a} | n.q.^{d} | **2c/3c** 56/44 |
| CYP_{BSß} | H₂O₂ | **1c** | 5 | 54^{a} | n.q.^{d} | **2c/3c** 64/36 |
| CYP_{BSß} | H₂O₂ | **1b** | 10 | 16 | 1.6 | **2b/3b** 76/24 |
| CYP_{BSß} | H₂O₂ | **1b** | 5 | 24 | 1.2 | **2b/3b** 84/16 |
| CYP_{BSß} | H₂O₂ | **1a** | 10 | 11 | 1.1 | **2a** >95%^{e} |
| CYP_{BSß} | H₂O₂ | **1a** | 5 | 16 | 08 | **2a** >95%^{e} |

| | | | | | | |
|---|---|---|---|---|---|---|
| ^{a}Based on substrate depletion. ^{b}5 % of product area was obtained for reaction with H₂O₂ and 10 mM of 1d, first entry. ^{c}outside of calibration range (only traces of substrate left). ^{d}n.q. = not quantified (reference compounds not available). ^{e} 3a-d not detected. | | | | | | |

Reaction conditions for P450_{Cla}: 3µM P450_{Cla}; Tris-HCl (pH 7.5; 0.1 M); 2.5 to 5 % EtOH (cosolvent for the substrate). 1.6 mM H₂O₂ was supplemented every hour (total 9.6 mM for 5 mM substrate and 19.2 mM for 10 mM substrate).

Reaction conditions for P450_{BSß}: 3µM P450_{BSß}; KPi buffer (pH 7.0; 0.1 M); 5 % EtOH (cosolvent). 1.6 mM H₂O₂ was supplemented every hour (total 8 mM for 5 mM substrate and 16mM for 10 mM substrate). All products were identified by GC-MS and/or comparison with authentic materials.

All reactions were performed in closed glass vials for 24 h reaction time at RT with 170 rpm shaking. The reactions were quenched by addition of 100 µl 5 N HCl. Extractions were performed with 500 µl EtOAc containing 0.1 % 1-decanol as internal standard. After drying over Na₂SO₄, derivatization of acids to corresponding methyl esters was realized by mixing 150 µl of organic phase with 90µl of MeOH prior to supplementation of 20 µl of TMSCHN₂ for esterification. Samples were analyzed by GC-FID and GC-MS. Commercial standards of 2-hydroxycaproic acid (2a), 2-hydroxyoctanoic acid (2b) and 2-oxooctanoic acid (4b) were used as references. Calibration curves were obtained from standards 1a-d and 2a-b prepared identically to the reaction mixtures (5 concentration points from 10 µM to 1 mM). Results show that P450_{Cla} is the better catalyst for α-hydroxylation of fatty acids with regards to regioselectivity (>95 % for 2a-d) and conversion (up to >99 %). Conversions tend to decrease with shorter chain length of the substrates, especially in case of CYP_{BSß} while P450_{Cla} prefers hydroxylation of dodecanoic acid 1d, especially at lower substrate concentration (5 mM).

### Step 2: Oxidation of α-hydroxy-acids

The second oxidation (dehydrogenation) was achieved by using two stereocomplementary 2-hydroxyisocaproate dehydrogenases (D-/L-HICDH) (scheme 2). Both enzymes were reported to catalyze the oxidation of 2a and 2b, however the reduction of *α*-keto-acids 4 was strongly favoured (>100-fold more efficient). The two HICDH enzymes were expressed and stored as lyophilized cell-free lysates until use. The results for the oxidation of α-hydroxy acids 2a-b are summarized in Table 3. In order to drive the reaction towards the thermodynamically unfavoured *α*-keto-products 4a-b, an NADH-oxidase (YcnD from *Bacillus subtillis*), which catalyzes the oxidation of NADH with formation of H₂O₂ as by-product, was employed in one reaction set-up to prove an internal NAD(P)+ regeneration system (scheme 2).

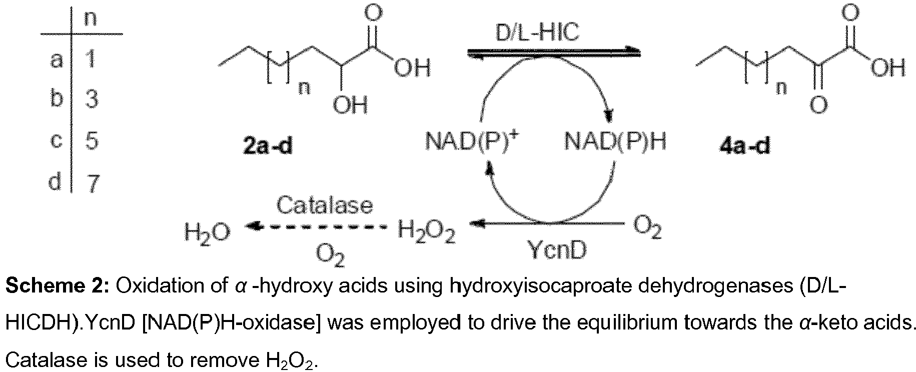

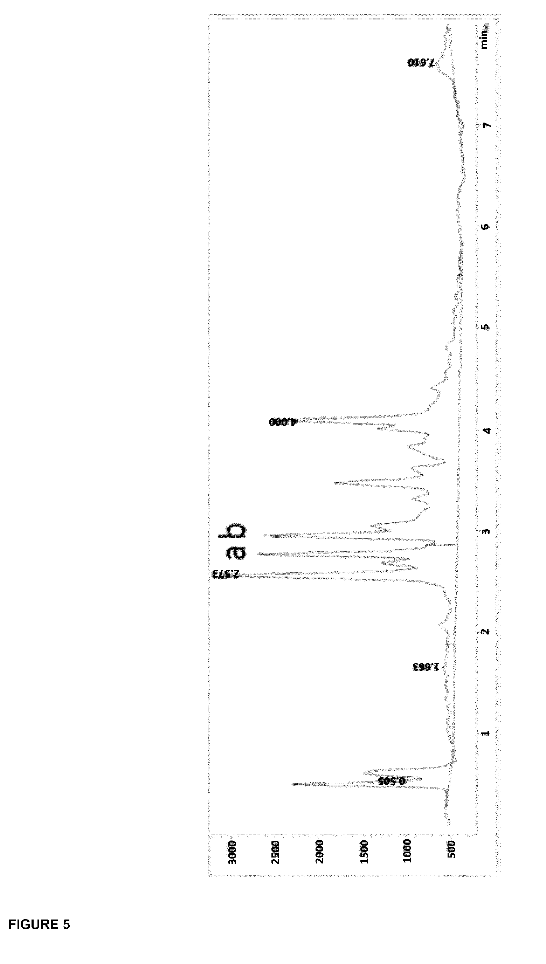

In first exploratory experiments (Table 3, the oxidation of α-hydroxy acids 2a/b to the respective *α-*keto acids 4a/b (Scheme 2) was not efficiently catalyzed by the D-/L-HIC proteins, but traces of *α-*keto-acid product 4b could be detected in presence of YcnD (Figure 5, A), indicating the unfavourable reaction equilibrium.

**Table 3 Oxidation/dehydrogenation of α-hydroxy acids 2a-b using D/L-HICDH catalysts.**

| **Catalysts** | **Substrate** | **α-Ketoacid 4a/b detected [Y/N]** |
|---|---|---|
| D/L-HICDH | 2b | No |
| D/L-HICDH-YcnD | 2b | Yes (traces) |
| YcnD | 2b | No |
| --- | 2b | No |
| D/L-HICDH | 2a | No |
| D/L-HICDH-YcnD | 2a | No |
| YcnD | 2a | No |

**Reaction conditions:** 10 mM Substrate (2a-b), 0.1 M KPi pH 7.5, 10 µl purified YcnD, 0.5 mg catalase (-600 U); D-/L-HICDH (5 mg each; lyophilized cell free lysate), 1 mM NAD+ (all reactions also performed with 6.75 mM); final volume 1 ml. Reactions were performed at RT with stirring at 170 rpm for 24 h.

Oxidation of the respective α-hydroxy acids 2a/b was also monitored spectrophotometrically by following the concomitant reduction of NAD+ via measurement of absorbance at 340 nm, however, no linear increase in absorbance could be detected. On the other hand, reduction of α-keto-acids 4a/b could be monitored by linear depletion of NADH, indicating that HICDH-proteins were active. Reduction of 2-oxo-octanoic acid 4b to 2-hydroxy octanoic acid 2b was also confirmed by GC-FID.

### EXAMPLE 3

### Three-step one-pot synthesis of norleucine from hexanoic acid

### Conversion of selected fatty acid substrates in cascade set-up

Hydroxylation of all four fatty acid substrates (C4, C6, C8, C10) was performed as described above using 3 µM P450_{Cla}, 10 mM substrate and 2.5 % EtOH in a final volume of 1 ml Tris-HCl (pH 7.5, 0.1 M). H₂O₂ was supplemented every 30 min (23 times; 36.8 mM total). After 24 h, the hydroxylation reaction (step 1) was stopped by adding 1200 U catalase (1 mg), 20µl of (NH₄)2SO₄(2.5 M stock) and the pH was adjusted to 8.5 using 1 M NaOH. For step 2 and 3 of the cascade, Land D-HICDH (0.25 mg), 10 mM NAD+ and 5 µl purified leucine-DH were added to the reaction mixture. After 24 h reaction time, 2 µl of the reaction volume were spotted on a TLC plate along with a commercial standard of L-norleucine as reference material (positive control). After elution of products acetic acid/H2O/n-butanol as mobile phase (1:1:3), the plate was stained with ninhydrin and heated gently for colour development. Only the sample from the enzyme cascade with hexanoic acid gave a distinct red spot after ninhydrin staining indicating the formation of norleucine. All other substrates did not display product (amino acid) formation. The following control reactions were performed to confirm the overall success of the two step cascade leaving one of the following components out of the reaction: 1) P450; 2) L- or D-HICDH; 3) Leu-DH; 4) fatty acid substrate; 5) H₂O₂; 6) (NH₄)2SO4; 7) NAD+. The reaction samples were analyzed by TLC as shown before, but formation of norleucine could not be detected.

### Derivatization and GC-MS analysis of cascade products

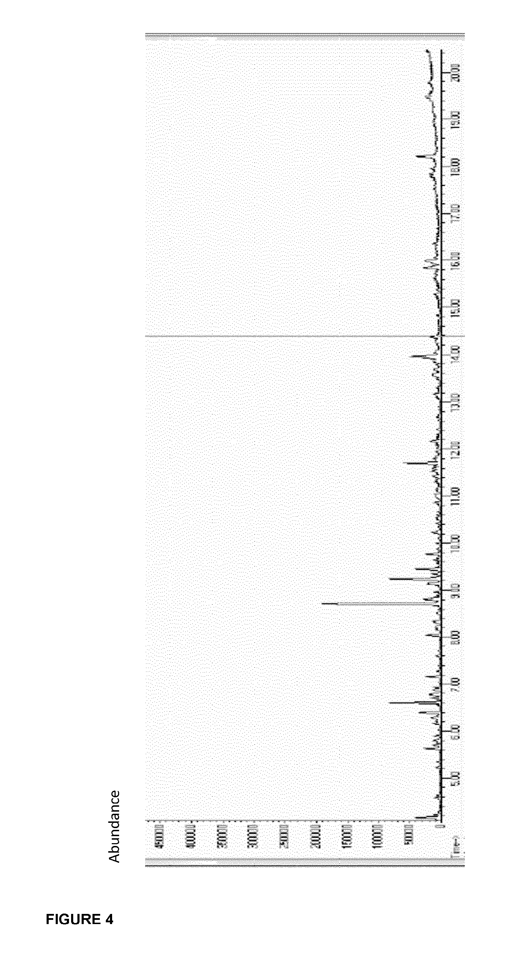

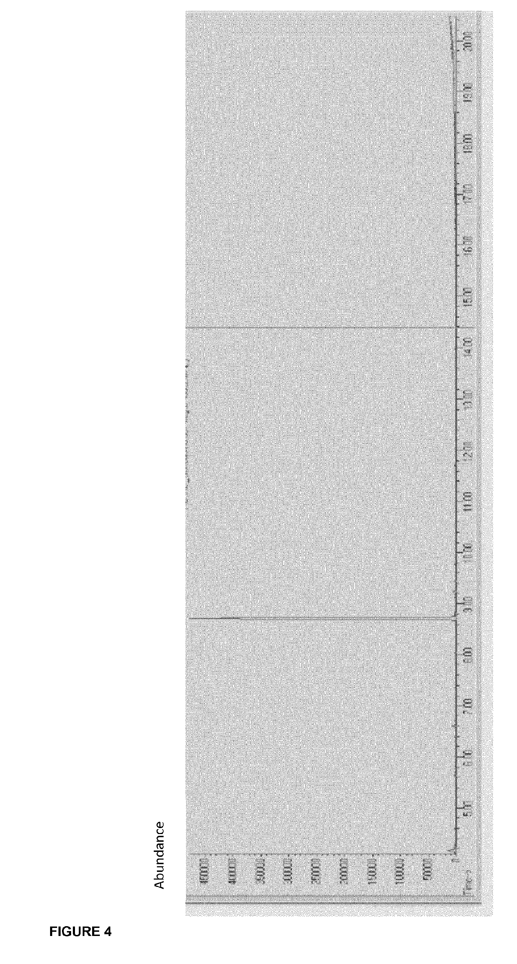

The reaction sample indicating formation of norleucine (positive ninhydrin staining on TLC plate) was freeze dried in liquid nitrogen and lyophilized overnight. Freeze dried residues were resuspended in 300 µl MeOH followed by addition of DMAP (5 % in 700 µl MeOH) and 150 µl ethyl chloroformate. The solution was stirred at 700 rpm for 50 min at 50 °C. MeOH was removed by evaporation (dry air flow) and 700 µl HCl solution (2 %) were added. The solution was extracted three times with 600 µl EtOAc and the organic phases were dried over anhydrous Na₂SO₄ prior to injection into GC-MS. In addition, 4 mg of L-norleucine (commercial standard) was treated in the same way and served as reference. The successful production of norleucine employing the three step one-pot cascade and hexanoic acid as substrate was confirmed by GC-MS (Figure 4).

### EXAMPLE 4

Alpha oxidation of 4-acetyloxy-butyric acid using the method disclosed in example 3. The results would include the intermediate alpha hydroxy acetyl butuyric acid as well as the product O-acetyl-L-homoserine.

### EXAMPLE 5

### Conversion of ethyl 4-acetoxybutanoate with P450 BM3

Ethyl acetoxy-butanoate (EAB) was converted with P450 BM3 variant M2 employing O₂ as oxidant.

### General methodology

The 3.1 kb P450 BM3 gene from *Bacillus megaterium* (CYP102A1) was cloned into the 2 kb pET22b-derived pALXtreme-1 a plasmid (Blanusa et al., 2011). For expression of the P450 BM3 monooxygenase ( EC 1.14.14.1) the pALXtreme-1, a plasmid, was transformed into chemical competent *E. coli* BL21 Gold (DE3)-derived E. coli laqI^{Q1} Gold (DE3) cells. Preparation of chemical competent cells was achieved following a standard protocol generating 108 clones per µξ pUC19 (Inoue et al., 1990). All plasmid preparations were performed using the QIAGEN QIAprep Spin Miniprep Kit (Hilden, Germany). All chemicals used were of analytical grade and purchased from Sigma Aldrich (Steinheim, Germany), abcr (Karlsruhe, Germany) or AppliChem (Darmstadt, Germany). Oligonucleotides used for mutagenesis were obtained from Eurofins MWG Operon (Ebersberg, Germany) and produced at HPLC purity. Enzymes were received from New England Biolabs (Frankfurt, Germany) and Sigma Aldrich (Steinheim, Germany). dNTPs were purchased from Fermentas (St. Leon-Rot, Germany).

For protein activity measurement of the P450 BM3 mutant libraries, frozen pellets of grown expression cultures stored for 4h at -20° C in 96-deep-well plates were resuspended by retro-pipetting in 150 µl 50 mM potassium phosphate buffer (KPi) pH 7.5. Additional 150 µl 50 mM KPi pH 7.5 was added containing 5 mg/ml lysozyme. Plates were incubated for 1 h at 900 rpm and 37°C in an Infors HT Microtron shaker to ensure entire cell lysis. Plates with lysed cells were centrifuged for 15 min at 3200 g and 4° C in an Eppendorf 581 OR centrifuge to spin down cell debris. The clear supernatant was used for assaying monooxygenase activity by measuring NADPH depletion at 340 nm in a Tecan Sunrise MTP reader (Groeding, Austria; Glieder & Meinhld, 2003). 96-well quarz glass MTPs (Hellma, Muellheim, Germany) were used during all measurements with p-xylene since the substrate reacts with most poly-propylene and poly-styrene based materials. A reaction mixture in MTP format contained 1.5 % DMSO, 200 mM p-xylene, 10 to 50 µl cell lysate and 0.16 mM NADPH in a final volume of 250 µl 50 mM KPi buffer pH 7.5. The amount of lysate had to be adjusted in a way that linear depletion of NADPH could be monitored reliably for 2 min. The reaction was stopped by pipetting 25 µl quenching buffer (4 M urea in 0.1 M NaOH) in the reaction mixture, followed by addition of 20 µl 4-aminoantipyrine (4-AAP) (5 mg/ml) and 20 µl potassium peroxodisulphate (5 mg/ml) for phenolic product quantification (Wong et al., 2005). After 30 min incubation at 750 rpm on an Eppendorf MixMate MTP shaker (Hamburg, Germany) the amount of 2.5-DMP was quantified at 509 nm absorbance in a Tecan Sunrise MTP reader (Wong et al.,supra). Mutants for next rounds of saturation were selected based upon best performance for NADPH depletion as well as highest formation of 2,5-DMP. Improved variants of P450 BM3 were sequenced at MWG Eurofins DNA (Ebersberg, Germany) and analyzed using Clone Manager 9 Professional Edition software (Scientific & Educational Software, Cary, NC, USA).

### Generation of P450 BM3 mutants

Three amino acid residues were chosen for iterative NNK saturation (R47, Y51, and I401). Amino acid residues R47 and Y51 were simultaneously saturated meanwhile site I401 was saturated as a single position. Oligonucleotides were designed for optimal PCR product yields according to recommendations of user's manual from Stratagene's QuikChange Site-directed mutagenesis Kit (Stratagene, La Jolla, CA, USA). A standard PCR set-up contained 20 ng plasmid DNA, 5 U Phusion DNA polymerase, 1 x Phusion DNA polymerase buffer, 0.2 mM dNTP mix and 0.4 µM of forward and reverse primer (Table 4). All PCRs were performed in thin wall PCR tubes (Saarstedt, Nuembrecht, Germany) and using an Eppendorf Mastercycler proS (Hamburg, Germany). The cycling protocol was performed according to Stratagene's QuikChange Site-directed mutagenesis Kit manual instruction (Stratagene, La Jolla, CA, USA). Temperatures and incubation times during each PCR cycle were adjusted to provider's recommendation for Phusion DNA polymerase (New England Biolabs, Frankfurt, Germany). Annealing temperature during each PCR cycle was adjusted to 55 °C for 30 s. Successful PCR amplifications were verified on 0.8% agarose gel according to a standard protocol (Maniatis et al. 1982). Digestion of template DNA was achieved by addition of 20 U Dpnl to 50 µl PCR mixture followed by incubation at 37°C for 3 h. The PCR products were transformed without further purification into chemically competent *E .coli* Gold (DE3) laqI^{Q1} cells using a heat shock transformation protocol (Hanahan et al., 1991). Recovered cells were plated and incubated overnight at 37° C on LB agar plates containing 100 Mg/ml kanamycin. Obtained colonies were transferred with sterile toothpicks into Greiner BioOne 96-well microtiterplates (MTP) (Frickenhausen, Germany) pre-filled with 100 µl LB media and 100 Mg/ml kanamycin. The plates were incubated for 14 h at 900 rpm and 37° C in an Infors HT Microtron shaker (Bottmingen, Switzerland) followed by addition of 100 µl of 50% glycerol (W/V). All MTPs were stored at -80° C prior to expression of mutant libraries.

**Table 4. Oligonucleotides used for NNK saturation of targeted amino acid residues.**

| Position | Primer name | SEQ ID No: | Primer sequence 5' - 3' |
|---|---|---|---|
| R47/Y51 | R47/Y51 Fw | 6 | CGAGGCGCCTGGTNNKGTAACGCGCNNKTTATCAAGTCAGCG |
| | R47/Y51 Rv | 7 | CGCTGACTTGATAAMNNGCGCGTTACMNNACCAGGCGCCTCG |
| I401 | I401 Fw | 8 | CAGCGTGCGTGTNNKGGTCAGCAGTTC |
| | I401 Rw | 9 | GAACTGCTGACCMNNACACGCACGCTG |

### Expression and purification of P450 BM3 mutants

The grown culture of the P450 BM3 mutant were centrifuged for 15 min at 2900 g and 4° C in an Eppendorf 5804R centrifuge (Hamburg, Germany) for removal of residual expression media. Obtained pellets were washed in 50 mM KPi pH 7.8 and spin down again applying the same centrifugation program (15 min; 2900 g; 4° C). The supernatant was removed and pellets were stored at -20° C for 12 h. For purification, frozen cell pellets were resuspended in 10 ml 50mM KPi buffer pH 7.8 prior to disruption in an Avestin EmulsiFlex-C3 high-pressure homogenizer (Ottawa, ON, Canada) by applying three cycles of 1500 bar pressure. Cell debris was removed by centrifuging homogenized samples for 30 min at 16000 g and 4° C in a Sorvall RC-6 Plus ultracentrifuge (Thermo Scientific, Rockford, IL, USA). Removal of small insoluble particles was achieved by pressing the supernatant through a 0.45 µM membrane filter. The filtered cell lysate was loaded on a Kronlab TAC15/ 125PE5-AB-2 column (YMC Europe GmbH, Dinslaken, Germany) prefilled with Toyopearl DEAE 650S anion exchange matrix (Tosoh Bioscience, Stuttgart, Germany). An AKTAprime Plus chromatography system with UV- detection (GE Healthcare, Muenchen, Germany) was used for purification and collection of eluted protein samples. A standard protocol was applied to purify P450 BM3 wild-type as well as the variant M2 (R47S/Y51W/I401M) (Schwaneberg et al.1999). Eluted protein samples were collected and purity was estimated on a 10% sodium dodecyl sulfate-polyacrylamide gel electrophoresis (SDS-PAGE). Fractions containing active and approximately 90% pure protein, were pooled in an Amicon Ultra-4 centrifugation tube (30 kDa cutoff; Millipore, Schwalbach, Germany) and concentrated to a final volume of 2 ml. Concentrated protein samples were desalted and equilibrated in 50 mM KPi pH 7.5 buffer using a PD-10 gel-filtration column (GE Healthcare, Muenchen, Germany). Finally enzyme samples were shock-frozen for long time storage in liquid N2 and lyophilized in an Alpha 1 -2 LD plus freeze-dryer (Christ, Osterode am Harz, Germany) for 48 h at -54° C.

The essential NADPH cofactor was regenerated using a glucose dehydrogenase (GDH) and glucose as shown in Scheme 3.

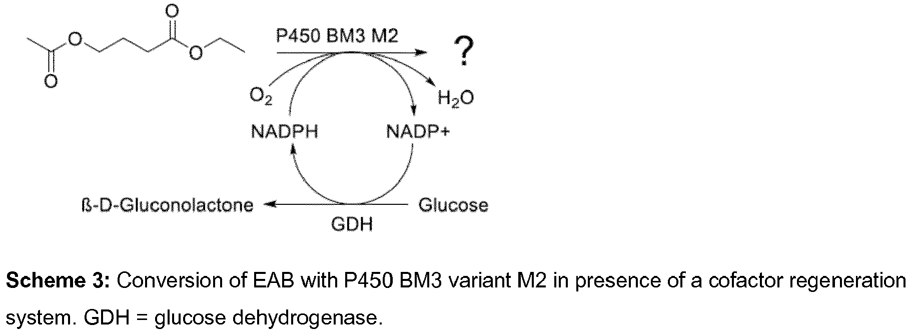

Reactions were done in 1 mL scale (KPi-buffer, pH 7.5, 0.1 M) containing following components:
12 U GDH
100 mM glucose
400 µM NADP+
50 mM EBA (substrate) in DMSO (5 % V/V DMSO final)
50 mg cell free freeze dried lysate of P450 BM3 variant M2

### Control reactions:

a) Empty vector; 50 mg of freeze dried *E. coli* BL21 (DE3) cells containing no P450 enzyme
b) Reaction containing no substrate (5 % V/V DMSO supplemented instead)

### GC-MS analysis

The product was recovered by liquid-liquid extraction using 500 µL EtOAc as organic phase. No product could be identified by GC-MS.

### LC-MS analysis

LC-MS analysis as formation of unstable (polar) intermediates/products occured (e.g. emiacetals), depending on the regioselectivity of the P450 monooxygenase catalyst (Scheme 4).

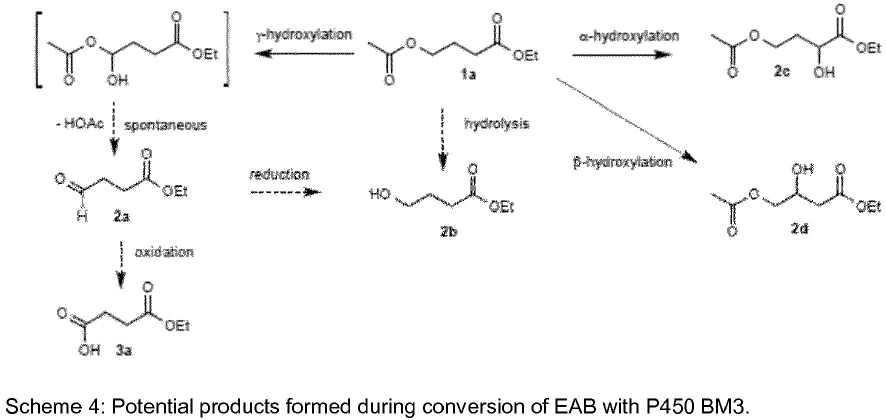

For analysis, 100 µl of the reaction volume were mixed with 100 µl acetonitrile followed by centrifugation to remove insoluble particles. The supernatant was diluted 1:50 in H2O/acetonitrile (50:50; V/V) followed by injection into the LC-MS.

To identify potentially formed side-products, LC-MS scans were done for following masses:
a) 130.06+1 = Product 2a
b) 146.06+1 = Product 3a
c) 132.08+1 = Product 2b
d) 190.08+1 = Product 2c and 2d
e) 174.09+1 = Substrate (EAB) 1a

### Mass-scanning of reaction samples

A mass-scanning was done to identify potential products formed during conversion of EAB with P450 BM3. At 2.96 min retention time a peak was detected that elutes before the substrate (retention time 3.699) indicating formation of a more polar compound. The same product peak was not detected in the control reaction which contains *E. coli* cells without P450 BM3.

### Analysis of reaction samples for mass 190.08+1 (target product 2c and 2d)

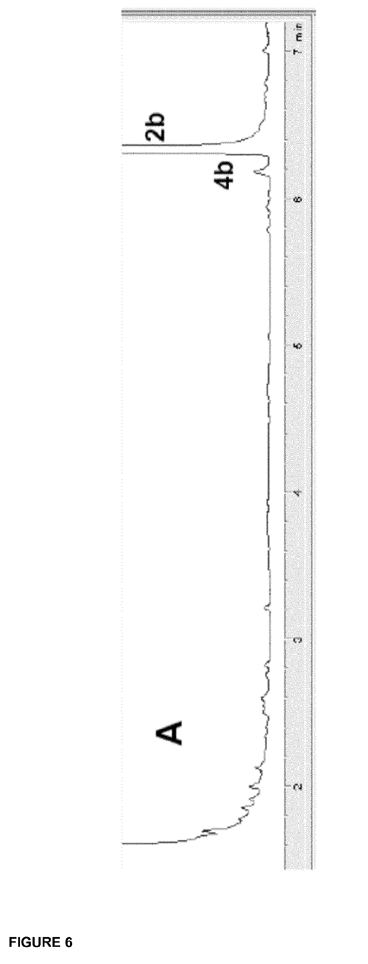

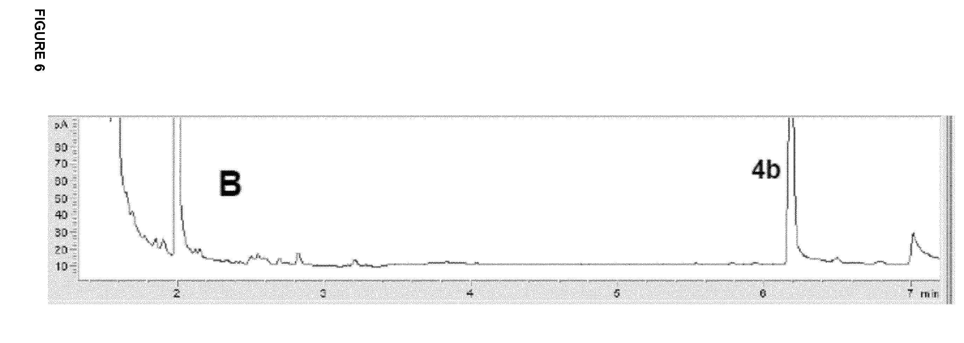

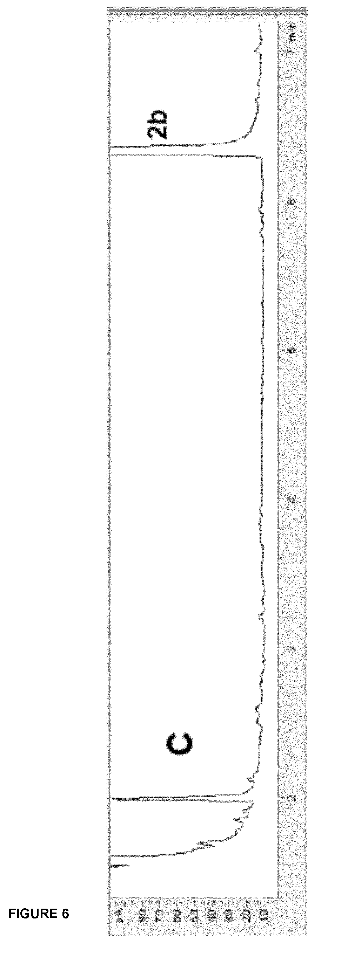

Detection of mass 190.08+1 revealed formation of two product peaks at 2.9 and 3.0 min that were only present in the reaction samples containing P450 BM3 variant M2 and EAB as substrate (Figure 6) Both peaks were not detectable in the control reactions nor in the substrate as possible contamination. In addition, the product peaks elute earlier than the substrate (3.699 min) indicating a higher polarity possibly due to a hydroxylation of EAB.

### Analysis of reaction samples for mass 132.08+1 (Product 2b) - potential hydrolysis of the acetoxygroup

A large peak signal was obtained for mass 132.06+1 (-20 % area) most likely corresponding to the formation of compound 2b (Scheme 4).

Formation of an unstable hemiacetal by P450 BM3 variant M2 is very unlikely since the same compound was also detected in the EV control reaction and no aldehyde formation occurred (2a, Scheme 1). Accordingly, intrinsic *E. coli* hydrolases were most likely responsible for the catalysing the formation of 2b.

P450 BM3 variant M2 possibly catalyses the formation of target 2c and unwanted side product 2d in rather equal amount. was hydrolyzed by *E. coli* endogeneous enzymes (e.g. esterases, lipases).

### REFERENCES

Abrahamson, M.J., Angew. Chem. Int. Ed. 2012, 51, 3969 -3972
A C Rosenzweig, et al., (1993) Nature, 366, 537-543
Adam, W. et al., (1996) Tetrahedron: Asymmetry, 7, 2287
Adam, W. et al., (1998) J. Am. Chem. Soc., 120, 11044
Blanusa et al. , Anal. Biochem 406, 141 -146, 2011
Getrey et. al., (2014) Green Chem., 16:1104
Girhard, M., et al., (2007) Biochem. Biophys. Res. Commun., 362, 114-119
Glieder & Meinhld, Methods Mol. Biol 230, Clifton, N.J. , USA, 157-170, 2003
Grant, C, et al. (2011) Enzyme and Microbial Technology, 480-486
Gutierrez, A., et al, (2011)Arch. Biochem. Biophys.; 514 (1-2): 33-43
H. Schütte, et al., (1984), Appl. Microbiol. Technol., 19, 167-176
Hanahan et al., Methods in enzymology 1991, 204, 63-1 13
Hwang and Kim (2004) Enzyme and Microbial Technology; 34: 429 - 436
Inoue et al., Gene 1990, 96, 23-28
J Sangster, Octanol-Water Partition Coefficients: Fundamentals and Physical Chemistry, Vol. 2 of Wiley Series in Solution Chemistry, John Wiley & Sons, Chichester, 1997
Kaulmann et al. (2007) Enzyme and Microbial Technology; 41: 628-637
Koch, D. J., et al., (2009)Appl. and Env. Microbiology, 337-344
Lo Piccolo, L, et al. (2011) Appl. Environm. Microbiol, 77 (4): 1204-12013
Malca S.H., et al (2011) Biochimica et Biophysica Acta 1814 257-264
Maniatis et al., 1982 1 - 545
Matsunaga M., Ukena K. and Tsutsui K. (2001) Brain Res. 899, 112-122
Nazor et al. (2008) Protein Engineering, Design & Selection; 21(1): 29-35
Nguyen, H. H. T., et al., (1998), J. Biol. Chem. 273, 7957-7966
Schwaneberg et al. J. Chromatogr. , A 1999, 848, 149-159.
W. Hummel, et al., (1985), Appl. Microbiol. Technol. 1985, 21, 7-15
Wong et al., J. Biomol. Screening 2005, 10, 246-252
Yun et al. (2005) Applied and Environmental microbiology; 4220-4224

## Claims

1. A microbial cell capable of producing at least one alpha amino acid from at least one short chain fatty acid, wherein the cell is genetically modified to comprise
- at least a first genetic mutation that increases the expression relative to the wild type cell of an enzyme E₁ capable of catalysing the hydroxylation of the fatty acid to an alpha-hydroxy fatty acid,
- at least a second genetic mutation that increases the expression relative to the wild type cell of an enzyme E₂ capable of catalysing the oxidation of the an alpha-hydroxy fatty acid to an alpha ketone, and
- at least a third genetic mutation that increases the expression relative to the wild type cell of enzyme E₃ capable of catalysing the oxidation of the alpha ketone to an alpha amino acid wherein the short chain fatty acid is a C₄- C₁₀ fatty acid.

2. The cell according to claim 1, wherein the enzyme E₁ is a cytochrome P450 enzyme (EC 1.14.x.x).

3. The cell according to claim 2, wherein the cytochrome P450 enzyme is P450_{Cla} (EC 1.11.2.4) or P450_{BSB} (EC 1.11.2.4).

4. The cell according to any one of the preceding claims, wherein the enzyme E₂ is a dehydrogenase selected from the group consisting of alpha-hydroxy acid oxidase (EC 1.1.1.337), alpha-hydroxy acid dehydrogenase (EC 1.1.1.345) and monooxygenase.

5. The cell according to any one of the preceding claims, wherein E₂ is selected from the group consisting of 2-hydroxyisocaproate dehydrogenase (EC 1.1.1.-), lactate dehydrogenase (EC. 1.1.1.28), lactate oxidase (EC 1.1.3.2), (S)-2-hydroxy-acid oxidase (EC 1.1.3.15), D- or L-mandelate oxidase (EC 1.1.3.X) and P450 monooxygenase (EC 1.14.x.x).

6. The cell according to any of the preceding claims, wherein the enzyme E₃ is selected from the group consisting of an amino acid dehydrogenase and alpha transaminase.

7. The cell according to any of the preceding claims, wherein the enzyme E₂ is 2-hydroxyisocaproate dehydrogenase (EC 1.1.1.-).and/or E₃ is leucine dehydrogenase (EC 1.4.1.9).

8. The cell according to any one of the preceding claims, wherein
- E₁ has 60% sequence identity with SEQ ID NO: 1 or 2,
- E₂ has 60% sequence identity with SEQ ID NO: 3 and/or SEQ ID NO: 5; and/or
- E₃ has 60% sequence identity with SEQ ID NO: 4.

9. The cell according to any one of the preceding claims, wherein the cell is a prokaryotic or a lower eukaryotic cell.

10. The cell according to any of the preceding claims, wherein the cell further comprises a reduced fatty acid degradation capacity relative to the wild type cell.

11. The cell according to claim 10, wherein the fatty acid degradation capacity is reduced by deletion of a gene encoding an enzyme selected from the group consisting of fatty acid importer, fatty acid-CoA ligase, acyl-CoA dehydrogenase, 2,4-dienoyl-CoA reductase, enoyl-CoA hydratase and 3-ketoacyl-CoA thiolase.

12. A method of producing at least one alpha amino acid from at least one short chain fatty acid, the method comprising
- contacting a recombinant microbial cell with a medium comprising the short chain fatty acid,
wherein the short chain fatty acid is a C₄- C₁₀ fatty acid; and
wherein the microbial cell is genetically modified to comprise
- at least a first genetic mutation that increases the expression relative to the wild type cell of an enzyme E₁ capable of catalysing the hydroxylation of the fatty acid to an alpha-hydroxy fatty acid,
- at least a second genetic mutation that increases the expression relative to the wild type cell of an enzyme E₂ capable of catalysing the oxidation of the an alpha-hydroxy fatty acid to an alpha ketone, and
- at least a third genetic mutation that increases the expression relative to the wild type cell of enzyme E₃ capable of catalysing the oxidation of the an alpha ketone to an alpha amino acid.

13. The method according to claim 12, wherein
- the enzyme E₁ is a monooxygenase;
- the enzyme E₂ is a dehydrogenase; and/or
- the enzyme E₃ is amino acid dehydrogenase or alpha transaminase.

14. The method according to any one of claims 12 to 13, wherein
- the enzyme E₁ is a cytochrome P450 enzyme;
- the enzyme E₂ is selected from the group consisting of alpha-hydroxy acid oxidase, alpha-hydroxy acid dehydrogenase and monooxygenase; and/or
- the enzyme E₃ is leucine dehydrogenase.

15. The method according to any one of claims 12 to 14 wherein the alpha amino acid is L-leucine.
